# EUROPEAN PATENT SPECIFICATION

(11) **EP 3 082 923 B1**
(45) Date of publication and mention of the grant of the patent: **08.05.2019**
(21) Application number: 14824070.8
(22) Date of filing: 17.12.2014
(51) Int. Cl.: A61M 16/04, A61M 16/01, A61M 16/00, A61M 16/08, A61M 39/08

(54) **FLEXIBLE AIRWAY DEVICE**
FLEXIBLE ATEMWEGVORRICHTUNG
DISPOSITIF RESPIRATOIRE SOUPLE

(30) Priority: 17.12.2013 GB 201322330
(43) Date of publication of application: 26.10.2016
(73) Proprietor: Intersurgical AG, Vaduz (LI); Nasir, Muhammed Aslam, Dr., Luton, Bedfordshire LU1 4AX (GB)
(72) Inventor: NASIR, Muhammed Aslam, Dr., Luton, Bedfordshire LU1 4AX (GB); KEMP, Jane Elizabeth, Bristol BS6 5DA (GB); MILLER, Andrew Neil, Crowthorne RG45 6DU (GB)
(74) Representative: CSY Herts
(86) International application number: PCT/GB2014/053745
(87) International publication number: WO 2015/092405

(56) References cited:
- WO-A1-91/12844
- WO-A1-2006/125986
- WO-A1-2009/142821
- WO-A2-2012/049448
- GB-A- 2 404 863
- US-A- 5 309 906
- US-A- 5 618 267
- US-A1- 2008 236 590
- US-A1- 2011 265 799
- US-A1- 2012 024 292
- US-B1- 7 762 261

## Description

### Field of the Invention

The present invention relates to medical devices and is applicable to airway devices comprising airway tubes. It is particularly applicable to supraglottic airway devices comprising airway tubes, more particularly to laryngeal airway devices comprising airway tubes and to their methods of manufacture. It is particularly applicable to airway devices, comprising airway tubes, used in the administration of Oxygen and/or anaesthetic gases to a human or veterinary patient breathing spontaneously, for Intermittent Positive Pressure Ventilation (IPPV) during a surgical procedure or resuscitation.

### Background to the Invention

GB2393399 (Nasir) describes an airway device comprising an airway tube having a first end and a second end, the first end of which is surrounded by a non-inflatable laryngeal cuff which forms an anatomical fit over the laryngeal inlet of a patient and a buccal cavity stabiliser located on or around the airway tube between the laryngeal cuff and the second end of the tube, the buccal stabiliser being adapted to prevent rotational or side-to-side movement of the airway device in use. In addition to the airway tube the airway device described may also comprise a gastric tube. The airway tube is provided for fluid connection with the lungs of the patient and the gastric tube is provided for fluid connection with the stomach of the patient. The gastric tube, if provided, is housed in the buccal cavity stabiliser next to the airway tube.

However, a buccal cavity stabiliser is not appropriate for all clinical situations and can sometimes impede rather than enhance the operation. For example in many opthamalogical, and maxillofacial or dental surgery the use of a reinforced tube is preferable, as the tube can flexibly move to one side to continue to provide an airway for the patient, whilst not interfering with the operation. An alternative device which does not have a buccal cavity stabiliser has been described in GB2404863.

In other supraglottic airway devices which do not have a buccal cavity stabiliser the airway tube and the gastric tube are typically formed as separate discrete tubes, which are then attached to one another such as described in EP1169077 (Brain). In addition both the airway tube and the gastric tube once formed are of fixed cross-sectional area. This limits the size of tubes, scopes and other devices that may need to be passed through the airway tube and the gastric tube to the fixed cross-sectional area of the respective tube. In order to accommodate larger tubes, scopes and other devices that may need to be passed through the airway tube and the gastric tube, attempts have been made in the prior art to provide airway devices having large bore airway tubes and the gastric tubes such as described in EP1220701 (Brain). However, such large bore airway tubes and the gastric tubes are also of fixed cross-sectional area.

US 2011/265799 (NELLCOR PURITAN BENNETT LLC) describes a tracheal tube having a flexible membrane disposed therein for separation of a ventilation lumen of the tracheal tube into multiple channels are provided. The flexible membrane is configured to divide a main ventilation lumen of the tracheal tube into an inspiration channel and an expiration channel. In some embodiments, a volume of the inspiration channel is substantially equal to a volume of the expiration channel.

US 2012/024292 (NELLCOR PURITAN BENNETT LLC) describes a tracheal tube having a shaped divider disposed therein for separation of a tracheal tube into multiple ventilation lumens are provided. In some embodiments, the divider divides a tracheal ventilation lumen from a bronchial ventilation lumen. In some embodiments, the shaped divider provides an irregular inner diameter that allows a relatively bulky device to be inserted into one or both lumens.

WO 2009/142821 (BOSTON SCIENT SCIMED INC) describes a system including an overtube configured to radially expand facilitates insertion of instruments in a patient. The system is directed to an overtube comprising an elongated body having a distal end and a proximal end, the overtube configured to radially expand from an insertion configuration to an expanded configuration, and including at least one articulation section having at least one degree of freedom and including a main channel expandable in the radial direction, and at least one additional channel. The at least one articulation section can radially expand. The system may include a radially expandable overtube having an optical channel configured to receive an optical device. The main channel and/or the at least one additional channel may be sized to receive one or more medical instruments.

WO 2006/125986 (LARYNGEAL MASK CO LTD) describes a laryngeal mask airway device for insertion into a patient to provide an airway passage to the patient's glottic opening, the device comprising an airway tube, a mask attached to the airway tube, the mask comprising a body having a distal end and a proximal end, a peripheral inflatable cuff, and defining an outlet for gas, the mask being connected to the airway tube for gaseous communication between the tube and the mask, the distal end of the mask being ventrally displaced, relative to the proximal end.

WO2012/019448 (LARYNGEAL MASK CO LTD; BRAIN ARCHIBALD IAN JEREMY) describes an artificial airway device to facilitate lung ventilation of a patient, comprising an airway tube, a gastric drain tube and a mask at one end of the airway tube, the mask including a backplate and having a peripheral formation capable of forming a seal around the circumference of the laryngeal inlet, the peripheral formation surrounding a hollow interior space or lumen of the mask and the airway tube opening into the lumen of the mask, wherein the mask includes an atrium for passage to the gastric drain tube of gastric matter leaving the oesophagus.

### Summary of the Invention

The present invention provides an airway device for human or animal use comprising an airway tube, according to claim 1. The dependent claims define preferred embodiments of the invention.

Also described herein is an airway tube for an airway device for human or animal use said airway tube having a first end and a second end, wherein the airway tube comprises a first lumen and a second lumen, each of said first and second lumens extending from the first end to the second end of the airway tube, wherein a flexible wall is provided between the first lumen and the second lumen extending from the first end to the second end of the airway tube, wherein movement of the flexible wall changes the cross-sectional area of the first lumen and the second lumen.

According to invention there is provided an airway device for human or animal use comprising and airway tube having a first end and a second end, the first end of which is surrounded by a laryngeal cuff configured to fit over the laryngeal inlet of a patient when in situ, wherein the airway tube comprises a first lumen and a second lumen, each of said first and second lumens extending from the first end to the second end of the airway tube, wherein a flexible wall is provided between the first lumen and the second lumen extending from the first end to the second end of the airway tube, wherein movement of the flexible wall varies the cross-sectional area of the first lumen and the second lumen wherein the first lumen is configured for airway access, and wherein the second lumen is configured for gastric access and wherein the second lumen is located wholly inside the first lumen and wherein the second lumen has a rest position, in which the second lumen is shaped to allow the second lumen to both collapse and to expand.

When the flexible wall is moved to increase the cross-sectional area of the first lumen this results in a decrease in the cross-sectional area of the second lumen.

When the flexible wall is moved to increase the cross-sectional area of the second lumen this results in a decrease in the cross-sectional area of the first lumen.

The first lumen is configured for airway access, preferably for fluid communication with the lungs of patient when in situ.

The second lumen is configured for gastric access, preferably for fluid communication with the stomach of the patient when in situ.

By providing an airway tube having two lumens for both airway access and gastric access, wherein the cross-sectional area of each can be varied, a greater variety of tubes, scopes and other devices that may be required to be passed through the respective lumens can be accommodated in a single airway tube, and thus a single airway device.

Preferably the circumference of the first lumen remains constant; however, the cross-sectional area is variable. Preferably the circumference of the first lumen is fixed; however, the cross-sectional area is variable. For the avoidance of doubt the term circumference is intended to include the cross-section length of the perimeter of the lumen, be it the first lumen or then second lumen.

In the alternative both the circumference and the cross-sectional area for the first lumen are variable.

Preferably the circumference of the second lumen remains constant; however, the cross-sectional area is variable. Preferably the circumference of the second lumen is fixed; however, the cross-sectional area is variable.

In the alternative both the circumference and the cross-sectional area for the second lumen are variable.

The first and second lumens may be formed separately as individual components. In this alternative the cross-sectional area of the second lumen is preferably smaller than the cross-sectional area of the first lumen. According to the invention, the second lumen is located wholly inside the first lumen. The second lumen in its rest position in cross-section is in the form of a deformable shape.

This means that when a large tube, scope or other instrument is passed down the first lumen it pushes against the deformable shape of the second lumen causing the second lumen to collapse and thus its cross-sectional area to decrease. This also means that when a large tube, scope or other instrument is passed down the second lumen it pushes against the deformable shape of the second lumen causing the second lumen to expand and thus its cross-sectional area to increase. The second lumen in its rest position is formed of any suitable deformable shape to allow the second lumen to both collapse (and thus its cross-sectional area to decrease) and to expand (and thus its cross-sectional area to increase). In one alternative the deformable shape is a lemon, in another alternative an inverted lemon, in another alternative a circle. In this alternative the wall of the second lumen forms the flexible wall provided between the first lumen and the second lumen. In this alternative the flexible wall may be expandable or non-expandable, such that the circumference of the second lumen is fixed or expandable.

Preferably the first lumen is configured to extend beyond the second lumen at the second end of the airway tube. In the alternative the second lumen is configured to extend beyond the first lumen at the second end of the airway tube.

Preferably the second lumen is configured to extend beyond the first lumen at the first end of the airway tube. In the alternative the second lumen is configured to extend beyond the first lumen at the first end of the airway tube.

Preferably the second end of the airway tube is provided with a connector. Preferably the connector is provided with a first lumen for fluid connection with the first lumen of the airway tube. Preferably the connector is provided with a second lumen for fluid connection with the second lumen of the airway tube.

Preferably the connector comprises a first component configured to fit over a portion of the second end of the airway tube. Preferably the first component is configured to provide an aperture to the second lumen of the connector and thus to the second lumen of the airway tube. Preferably the aperture is provided in the side wall of the first component. Preferably the connector comprises a second component configured to fit inside a portion of the second end of the first lumen such that a portion of the second end of the first lumen is retained between the first and second components of the connector to retain the connector in position about the airway tube. Preferably the portion of the second end of the first lumen is pinched or squashed between the first and second components of the connector. Preferably the first and second components of the connector are provided with respective male and female interlocking components to interlock the first and second components of the connector. The male and female interlocking components may cooperate for example to comprise a spring clip.

Preferably the external surface of the airway tube is provided with a reinforcing means. Preferably the reinforcing means comprises a spiral bead of material of greater shore hardness than the airway tube located around the external wall of the airway tube. Preferably the reinforcing means extends from the second end of the airway tube to the first end of the airway tube. Preferably the reinforcing means comprises polypropylene.

The reinforcing means is used to prevent kinking of the airway tube when it is bent. A kinked airway tube can result in the air supply to the patient being significantly reduced or cut off.

In the prior art the reinforcing means is typically a spiral wire embedded into the wall of the airway tube. However, by providing an external reinforcing means from a plastics material this means that the device can remain in situ in the patient during procedures where metal cannot be used such as in Magnetic Resonance Imaging (MRI) procedures.
Preferably the airway tube is formed via linear extrusion with a rotating extruder ring around configured to extrude the spiral bead whilst the airway tube linear extrusion is still hot.

Preferably the reinforcing means does not extend beyond the first lumen at the first end of the airway tube.

Preferably the reinforcing means does not extend beyond the second lumen at the second end of the airway tube.

Preferably the cuff is non-inflatable and is pre-formed in a shape adapted to form an anatomical fit over the laryngeal framework of a patient.

Preferably the laryngeal cuff is pre-formed, pre-inflated with air or pre-filled with a suitable fluid. Most preferably the laryngeal cuff is non-inflatable, however in the alternative the laryngeal cuff can be inflatable.

Preferably the laryngeal cuff is over moulded onto the first end of the airway tube linear extrusion.

Preferably the second lumen is configured to exit the laryngeal cuff at the tip thereof.

In one alternative the airway device further comprises a buccal cavity stabiliser located on or around the airway tube between the laryngeal cuff and the second end of the tube. The buccal cavity stabiliser, if provided, may be formed from the same material as the cuff or from a different material and assists in locating and maintaining the position of the device in use.

In a particularly preferred embodiment the buccal cavity stabiliser, if provided, is formed as an integral part of the airway tube, and further preferably the buccal cavity stabiliser, if provided, the airway tube and the laryngeal cuff are all formed as an integral unit.

In a further alternative no buccal cavity stabiliser is provided.

The Shore hardness of the various, parts, portions or components is an important feature of the invention. For example, the laryngeal cuff is preferably formed from a material with a Shore hardness on the A scale of 40 or less and more preferably 000 to 20, and most preferably 000 to 4.

Preferably the laryngeal cuff and a front, ventral part of the buccal cavity stabiliser, if provided, are formed from a material of substantially the same Shore hardness. This simplifies construction and ensures that all portions of the device that come into firm contact with the patient's soft tissue are relatively soft.

In a further preferred embodiment a back or dorsal part of the device and a front or ventral part of the device are formed from materials of different Shore hardness. This enables the dorsal portion to be made of a firmer material than the ventral portion.

Preferably the back or dorsal part of the device is formed from a material of Shore hardness less than 60 on the A scale, more preferably 25 to 45, and most preferably 30 to 40.

### Brief Description of the Drawings

The invention will now be described, by way of example only, with reference to the accompanying drawings in which:-
Figure 1 illustrates a cross-sectional view of the airway tube according to a comparative example not falling within the scope of the invention;
Figure 2 illustrates a perspective view of a portion of the airway tube according to a comparative example not falling within the scope of the invention;
Figure 3 illustrates a side view of the airway tube according to a comparative example not falling within the scope of the invention;
Figure 4 illustrates a cross-sectional view of the airway tube of an airway device according to an embodiment of the invention;
Figure 5 illustrates a cross-sectional view of the second lumen of the airway tube of an airway device according to an embodiment of the invention;
Figure 6 illustrates a perspective view of a portion of the airway tube of an airway device according to an embodiment of the invention;
Figure 7 illustrates a longitudinal cross-sectional view of the airway tube according to a comparative example not falling within the scope of the invention, in combination with a laryngeal cuff;
Figure 8a illustrates a side view of the second portion of the airway tube according to a comparative example not falling within the scope of the invention, with a connector;
Figure 8b illustrates a side cross-sectional view of the second portion of the airway tube according to a comparative example not falling within the scope of the invention, with a connector; and
Figure 9 illustrates a cross-sectional view of an alternative construction of the second lumen of the airway tube of an airway device according to an embodiment of the invention.

### Description of the Preferred Embodiments and Comparative Examples

Embodiments of the present invention, and comparative examples not falling within the scope of the invention, are described below by way of example only. The embodiments of the invention described below represent the best ways of putting the invention into practice that are currently known to the applicant although they are not the only ways in which this could be achieved.

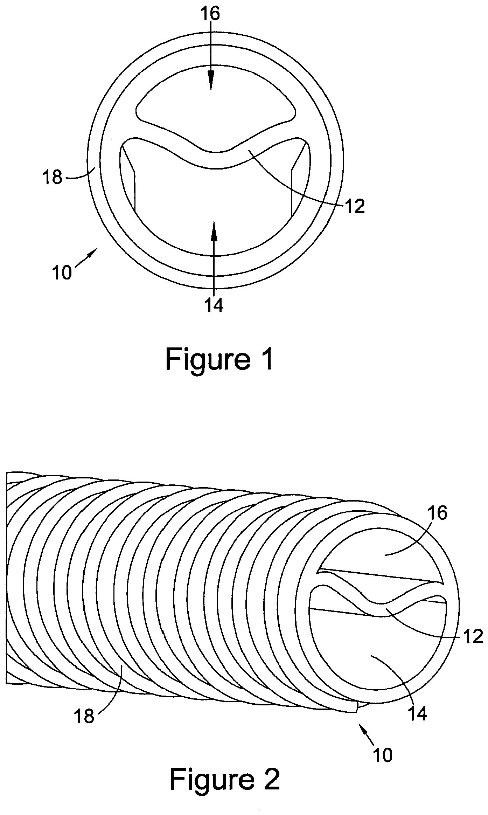

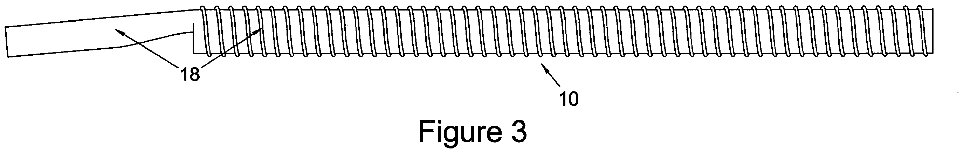

Referring to Figures 1 to 3, these illustrates an airway tube 10 according to a comparative example not falling within the scope of the invention. The airway tube 10 has a first lumen 14, a second lumen 16 and a flexible wall 12 there between. The first lumen 14 is configured for airway access, and delivers gasses to the patient. The second lumen 16 is configured for gastric access. It may be necessary after the airway tube 10 has been inserted into the patient to insert further tubes, scopes or other devices through either the first lumen 14, or the second lumen 16, into the patient. When a tube needs to be inserted into the first lumen 14, that is larger than the cross-sectional area illustrated in Figure 1, the flexible wall 12 simply moves, as the tube, is inserted, towards the second lumen 16. In doing so the cross-sectional area of the first lumen 14 increases, and the cross-sectional area of the second lumen 16 decreases to accommodate the tube through the first lumen. Should a tube then need to be inserted into the second lumen 16, the tube inserted into the first lumen 14 can be removed, and then the tube which is larger in cross-section than the current cross-sectional area of the second lumen 16, can then be inserted into the second lumen 16. As the tube is inserted into the second lumen 16, the flexible wall 12 again simply moves, as the tube is inserted, towards the first lumen 14. In doing so the cross-sectional area of the second lumen 16 increases and the cross-sectional area of the first lumen 14 decreases, to accommodate the tube through the second lumen 16.
In the alternative, as well as the flexible wall 12 being flexible, the flexible wall 12 is also expandable, such that not only the cross-sectional area of the respective lumens 14, 16 changes, but also the circumference thereof.

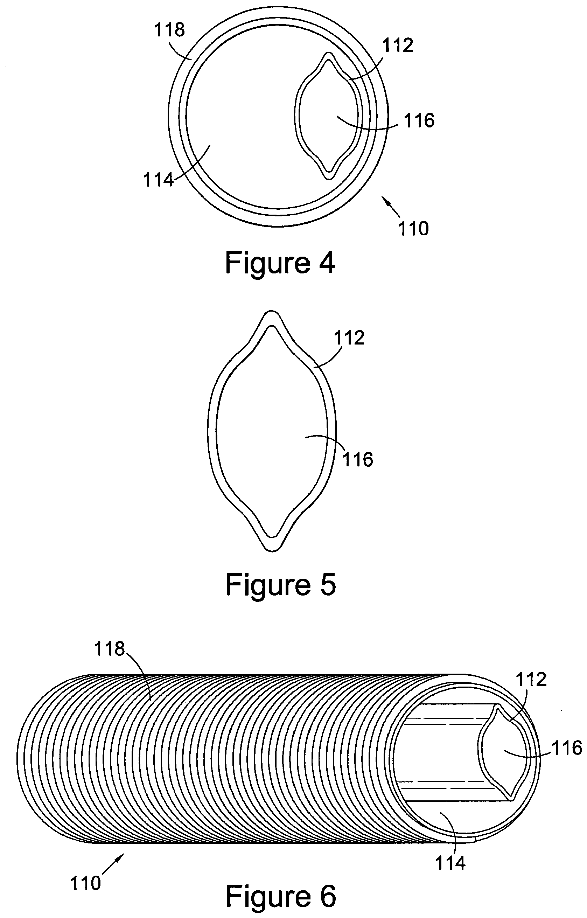

Referring to Figures 4 to 6, these illustrate an airway tube 110 of an airway device according to an embodiment of the invention. The airway tube 110 has a first lumen 114 within which is housed a second lumen 116. The wall 112 of the second lumen forms the flexible wall there between. The first lumen 114 is configured for airway access, and delivers gasses to the patient. The second lumen 116 is configured for gastric access. When a tube needs to be inserted into the first lumen 114, that is larger than the cross-sectional area illustrated in Figure 4, the flexible wall 112 simply moves, as the tube, is inserted, to cause the second lumen 116 to collapse. In doing so the cross-sectional area of the first lumen 114 increases, and the cross-sectional area of the second lumen 116 decreases to accommodate the tube through the first lumen. Should a tube then need to be inserted into the second lumen 116, the tube inserted into the first lumen 114 can be removed, and then the tube which is larger in cross-section than the current cross-sectional area of the second lumen 116, can then be inserted into the second lumen 116. As the tube is inserted into the second lumen 16, the flexible wall 112 again simply moves, as the tube is inserted, to cause the second lumen 116 to expand. In doing so the cross-sectional area of the second lumen 116 increases and the cross-sectional area of the first lumen 114 decreases, to accommodate the tube through the second lumen 116.

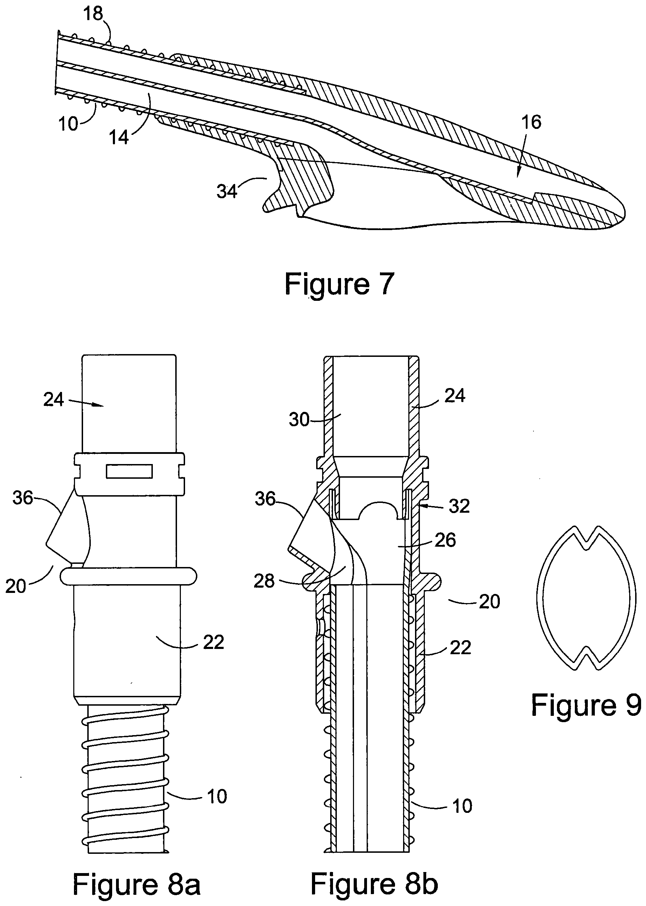

In the embodiment illustrated the shape of cross-section of the second lumen 116 is a lemon shape. Such a shape allows the second lumen 116 to readily collapse and expand. Other suitable shapes of cross-section of the second lumen 116 which also allow the second lumen 116 to readily collapse and expand include an inverted lemon wherein the tips of the lemon point into rather than away from the body of the lemon as illustrated in Figure 9. Further in the alternative the cross-section of the second lumen 116 may be a circle. Yet further in the alternative the cross-section of the second lumen 116 may be any other suitably deformable shape.

In the alternative, as well as the flexible wall 112 being flexible, the flexible wall 112 is also expandable, such that not only the cross-sectional area of the respective lumens 114, 116 changes, but also the circumference of the second lumen 116.

In both the above described embodiment and comparative example, the second lumen 16, 116 optionally extends beyond the first lumen 14, 114 at the second end of the airway tube 10, 110 and the second lumen 16, 116 optionally extends beyond the first lumen 14, 114 as the first end of the airway tube 10, 110.

In both the above described embodiment and comparative example, the external surface of the airway tube 10, 110 is optionally provided with a reinforcing means 18, 118. The reinforcing means 18, 118 is configured to prevent the airway tube from kinking when it is bent. The reinforcing means 18, 118 illustrated in accordance to the first and second embodiments is a spiral bead of material, in one alternative a plastics material, of greater shore hardness than the airway tube 10, 110 itself such as polypropylene.

Where a reinforcing means 18, 118 is provided it optionally does not extend beyond the end of the second lumen 16, 116 at the second end of the airway tube 10, 110 and the end of the first lumen 14, 114 at the first end of the airway tube 10, 110.

In both the above described embodiment and comparative example, the airway tube 10, 110 is optionally provided with a connector 20 configured to connect the airway tube 10, 110 for connection to an anaesthetic breathing system of conventional type. A suitable connector 20 is illustrated in Figures 8a and 8b.

The connector 20 is formed from a first component 22 and a second component 24. The first component 22 is provided with a first lumen 26 for fluid connection with the first lumen 14 of the airway tube 10 and a second lumen 28 for fluid connection with the second lumen 16 of the airway tube 10. The first component 22 is also provided with an aperture 36 in the side wall thereof for fluid connection with the second lumen 28 of the first component 22 and thus the second lumen 16 of the airway tube 10. The second component 24 is provided with a first lumen 30 for fluid connection with the first aperture 26 of the first component 22 and the first lumen 14 of the airway tube 10.

The first component 22 of the connector 20 is configured to fit over a portion of the second end of the airway tube 10. In the arrangement illustrated in Figures 8a and 8b, the first lumen 14 of the airway tube 10 is configured to extend beyond the second lumen 16 of the airway tube 10 at the second end of the airway tube. The first component 22 of the connector 20 is configured to fit over a portion of the second end of the first lumen 14 of the airway tube 10. The second aperture 28 of the first component 22 is configured to connect with the second lumen 16 of the airway tube 10 and exit the side wall of the first component 22.

The second component 24 of the connector 20 is configured to fit inside a portion of the second end of the airway tube 10. In the arrangement illustrated in Figures 8a and 8b, the first lumen 14 of the airway tube 10 is configured to extend beyond the second lumen 16 of the airway tube 10 at the second end of the airway tube. The second component 24 of the connector 20 is configured to fit inside a portion of the second end of the first lumen 14 of the airway tube 10 such that a portion of the first lumen 14 is retained between the first and second components 22, 24 of the connector 20 to retain the connector 20 in position about airway tube 10.

The first lumen 14 should be pinched or squashed between the first and second components 22, 24 of the connector 20 to retain the connector 20 in position about airway tube 10.

In order to connect the first and second components 22, 24 together to form the connector 20 once it is in situ about the airway tube 10 the first and second components 22, 24 are provided with interlocking male and female components to interlock the first and second components 22, 24 of the connector 20. In the arrangement illustrated in Figures 8a and 8b a spring clip 32 is provided.

In one arrangement illustrated in Figure 7 formed around the first end of the airway tube is a laryngeal cuff 34. In the arrangement illustrated the laryngeal cuff is non inflatable and is adapted in its shape and contours to correspond with the laryngeal inlet region of a patient. Preferably, the laryngeal cuff is non-inflatable and is formed from any suitable soft plastics material. By way of a preferred softness (hardness) range, on the Shore A scale of Hardness, a hardness of less than 40 for the face of the laryngeal cuff that contacts the laryngeal inlet is optimum. By way of a preferred range, a value on the same scale of 000 to 20 is preferred, with a particularly preferred range of 000 to 4. The softness of the laryngeal cuff can be further adapted by forming cavities or channels within the body of the cuff itself.

Preferably, the laryngeal cuff may be pre-filled with a fluid such as air, or other non-toxic gas, or a non-toxic liquid. In this context the term fluid has a broad meaning and includes any suitable gas, liquid, vapour or combination thereof and will be determined and designed by an expert in this field of anatomy/anaesthesia in conjunction with the materials specialist. The laryngeal cuff will be constructed of such a material which will not allow nitrous oxide (anaesthetic gas) to diffuse through the material to any significant amount so that the extra luminal pressure is kept constant. It follows therefore that the laryngeal cuff should be substantially impermeable to the fluid with which is filled and to anaesthetic gases.

Alternatively, the laryngeal cuff can be formed from a soft, foamed material or can be foam filled. In either case this provides a soft deformable but shaped surface around the face of the laryngeal cuff to engage over the anatomy of the larynx inlet region. Such a foam filled device will minimise any potential damage to the structures in that region whilst still providing a substantially complete seal.

Further in the alternative the laryngeal cuff is pre-filled during manufacture with a fluid in which case the lining of the cuff should be made from a material that does not absorb anaesthetic gases such as Nitrous Oxide, such that the pressure inside the cuff does not rise during use.

Alternatively, the laryngeal cuff may be formed from a material which is adapted to absorb a liquid, such as water, mucous or blood or similar liquid material and in doing so to swell in size so as to confirm to the anatomical mucocartilagenous framework of the patient's laryngeal inlet. Such materials will be selected by the materials specialist but include CRM (cotton rayon mixes) as used in TAMPAX (RTM) tampons, or compressed Gel Foam 5.

Alternatively, the laryngeal cuff could take the form of a conventional, inflatable laryngeal cuff. The technology to form an inflatable laryngeal cuff is well known and need not be described here.

Finally the laryngeal cuff may be hollow, but not inflatable in the traditional sense of the word, and instead Positive Pressure Ventilation is employed to "inflate" and self-pressurise the laryngeal cuff.

The device may be constructed from any suitable plastics material as selected by the materials specialist. Latex-free medical grade silicone rubber is one preferred material. The cuff should be soft in texture to avoid undue damage to the surrounding tissue. Other suitable materials for construction of this type of device include, but are not limited to, Poly Vinyl Chloride (PVC), Thermoplastic Elastomers such as the styrenic block copolymers (eg Styrene Butadiene Styrene (SBS), Styrene Ethylene Butylene Styrene (SEBS)), and Thermoplastic Olefin Blends (TPO), Thermoplastic PolyUrethanes (TPU), Copolyester (COPE), Polyether Block Amides (PEBAX) and foamed versions thereof, where appropriate.

A further important factor involved in the choice of a suitable material is transparency. Ideally the material or materials of construction should be substantially clear or transparent. This enables the anaesthetist or operator to see the inner lumen of the airway to check for blockages or other problems. Such transparent materials are known to the materials specialist.

## Claims

1. An airway device for human or animal use comprising an airway tube (110), said airway tube having a first end and a second end, the first end of which is surrounded by a laryngeal cuff (34) configured to fit over the laryngeal inlet of a patient when in situ, wherein the airway tube comprises a first lumen (114) and a second lumen (116), each of said first and second lumens extending from the first end to the second end of the airway tube, wherein a wall (112) is provided between the first lumen (114) and the second lumen (116) extending from the first end to the second end of the airway tube, wherein the first lumen (114) is configured for airway access, and wherein the second lumen (116) is configured for gastric access and wherein the second lumen (116) is located wholly inside the first lumen (114),
**characterised in that** the wall (41) is flexible,
**in that** movement of the flexible wall (41) varies the cross-sectional area of the first lumen (114) and the second lumen (116), and
**in that** the second lumen (116) has a rest position, in which the second lumen (160) is shaped to allow the second lumen (116) to both collapse and to expand.

2. An airway device as claimed in claim 1 wherein movement of the flexible wall (112) to increase the cross-sectional area of the first lumen (114) results in a decrease in the cross-sectional area of the second lumen (116).

3. An airway device as claimed in claim 1 or claim 2 wherein movement of the flexible wall (112) to increase the cross-sectional area of the second lumen (116) results in a decrease in the cross-sectional area of the first lumen (114).

4. An airway device as claimed in any preceding claim wherein the first lumen (114) has a circumference and wherein the device is configured such that the circumference of the first lumen (114) remains constant.

5. An airway device as claimed in any preceding claim wherein the second lumen (116) has a circumference and wherein the device is configured such that the circumference of the second lumen (116) remains constant.

6. An airway device as claimed in any preceding claim wherein the first (114) and second (116) lumens are formed separately as individual components.

7. An airway device as claimed in any preceding claim wherein the second end of the airway tube (110) is provided with a connector (20) wherein the connector comprises a first component (22) configured to fit over a portion of the second end of the airway tube and a second component (24) configured to fit inside a portion of the second end of the first lumen (114) such that a portion of the second end of the first lumen (114) is retained between the first (22) and second (24) components of the connector (20) to retain the connector (20) in position about the airway tube (110).

8. An airway device as claimed in any preceding claim wherein the external surface of the airway tube (110) is provided with a reinforcing means (118).

## Patentansprüche

1. Atemwegsvorrichtung zur Verwendung durch einen Menschen oder ein Tier, wobei die Vorrichtung ein Atemwegsrohr (110) umfasst,
wobei das Atemwegsrohr ein erstes Ende und ein zweites Ende aufweist, wobei das erste Ende von einer Kehlkopfmanschette (34) umgeben ist, die so gestaltet ist, dass sie in situ über den Kehlkopfeingang eines Patienten passt, wobei das Atemwegsrohr ein erstes Lumen (114) und ein zweites Lumen (116) umfasst, wobei sich das erste Lumen und das zweite Lumen jeweils von dem ersten Ende zu dem zweiten Ende des Atemwegsrohrs erstrecken, wobei zwischen dem ersten Lumen (114) und dem zweiten Lumen (116) eine Wand (112) bereitgestellt ist, die sich von dem ersten Ende zu dem zweiten Ende des Atemwegsrohrs erstreckt;
wobei das erste Lumen (114) für einen Atemwegszugang gestaltet ist, und wobei das zweite Lumen (116) für einen Magenzugang gestaltet ist, und wobei sich das zweite Lumen (116) vollständig in dem ersten Lumen (114) befindet,
**dadurch gekennzeichnet, dass** die Wand (41) flexibel ist, wobei eine Bewegung der flexiblen Wand (41) die Querschnittsfläche des ersten Lumens (114) und des zweiten Lumens (116) variiert, und wobei das zweite Lumen (116) eine Ruhestellung aufweist, an welcher das zweite Lumen (160) so geformt ist, dass es es ermöglicht, dass das zweite Lumen (116) sowohl zusammenfällt als auch sich erweitert.

2. Atemwegsvorrichtung nach Anspruch 1, wobei eine Bewegung der flexiblen Wand (112) zur Vergrößerung der Querschnittsfläche des ersten Lumen (114) zu einer Reduzierung der Querschnittsfläche des zweiten Lumen (116) führt.

3. Atemwegsvorrichtung nach Anspruch 1 oder 2, wobei eine Bewegung der flexiblen Wand (112) zur Vergrößerung der Querschnittsfläche des zweiten Lumen (116) zu einer Reduzierung der Querschnittsfläche des ersten Lumen (114) führt.

4. Atemwegsvorrichtung nach einem der vorstehenden Ansprüche, wobei das erste Lumen (114) einen Umfang aufweist, und wobei die Vorrichtung so gestaltet ist, dass der Umfang des ersten Lumen (114) konstant bleibt.

5. Atemwegsvorrichtung nach einem der vorstehenden Ansprüche, wobei das zweite Lumen (116) einen Umfang aufweist, und wobei die Vorrichtung so gestaltet ist, dass der Umfang des zweiten Lumen (116) konstant bleibt.

6. Atemwegsvorrichtung nach einem der vorstehenden Ansprüche, wobei das erste (114) und das zweite (116) Lumen separat als einzelne Komponenten ausgebildet sind.

7. Atemwegsvorrichtung nach einem der vorstehenden Ansprüche, wobei das zweite Ende des Atemwegsrohrs (110) mit einem Verbinder (20) versehen ist, wobei der Verbinder eine erste Komponente (22) umfasst, die so gestaltet ist, dass sie über einen Teil des zweiten Endes des Atemwegsrohrs passt, und eine zweite Komponente (24), die so gestaltet ist, dass sie in einen Teil des zweiten Endes des ersten Lumens (114) passt, so dass ein Teil des zweiten Endes des ersten Lumen (114) zwischen der ersten (22) und der zweiten (24) Komponente des Verbinders (20) gehalten wird, um den Verbinder (20) an der Position um das Atemwegsrohr (110) zu halten.

8. Atemwegsvorrichtung nach einem der vorstehenden Ansprüche, wobei die äußere Oberfläche des Atemwegsrohrs (110) mit einem Verstärkungsmittel (118) versehen ist.

## Revendications

1. Dispositif respiratoire à usage humain ou animal comprenant un tube respiratoire (110),
ledit tube respiratoire ayant une première extrémité et une seconde extrémité, la première extrémité étant entourée par un brassard laryngé (34) conçu pour s'adapter sur l'entrée laryngée d'un patient lorsqu'il est in situ, le tube respiratoire comprenant une première lumière (114) et une seconde lumière (116), chacune desdites première et seconde lumières s'étendant de la première extrémité à la seconde extrémité du tube respiratoire, une paroi (112) étant disposée entre la première lumière (114) et la seconde lumière (116) s'étendant de la première extrémité à la seconde extrémité du tube respiratoire,
la première lumière (114) étant conçue pour l'accès aux voies respiratoires, et la seconde lumière (116) étant conçue pour l'accès gastrique et la seconde lumière (116) étant située entièrement à l'intérieur de la première lumière (114),
**caractérisé en ce que** la paroi (41) est flexible,
**en ce que** le mouvement de la paroi flexible (41) fait varier la surface de la section transversale de la première lumière (114) et de la seconde lumière (116), et
**en ce que** la seconde lumière (116) a une position de repos, la seconde lumière (160) étant formée pour permettre à la seconde lumière (116) de s'affaisser et de se dilater.

2. Dispositif respiratoire selon la revendication 1, le mouvement de la paroi flexible (112) pour augmenter la surface de la section transversale de la première lumière (114) entraînant une diminution de la surface de la section transversale de la seconde lumière (116).

3. Dispositif respiratoire selon la revendication 1 ou 2, le mouvement de la paroi flexible (112) pour augmenter la surface de la section transversale de la seconde lumière (116) entraînant une diminution de la surface de la section transversale de la première lumière (114).

4. Dispositif respiratoire selon l'une quelconque des revendications précédentes, la première lumière (114) ayant une circonférence et le dispositif étant conçu de sorte que la circonférence de la première lumière (114) reste constante.

5. Dispositif respiratoire selon l'une quelconque des revendications précédentes, la seconde lumière (116) ayant une circonférence et le dispositif étant conçu de sorte que la circonférence de la seconde lumière (116) reste constante.

6. Dispositif respiratoire selon l'une quelconque des revendications précédentes, les première (114) et seconde (116) lumières étant formées séparément en tant que composants individuels.

7. Dispositif respiratoire selon l'une quelconque des revendications précédentes, la seconde extrémité du tube respiratoire (110) étant munie d'un raccord (20), le raccord comprenant un premier composant (22) conçu pour s'adapter sur une partie de la seconde extrémité du tube respiratoire et un second composant (24) conçu pour s'adapter à l'intérieur d'une partie de la seconde extrémité de la première lumière (114) de sorte qu'une partie de la seconde extrémité de la première lumière (114) soit retenue entre les premier (22) et second (24) composants du connecteur (20) pour maintenir le raccord (20) en position autour du tube respiratoire (110).

8. Dispositif respiratoire selon l'une quelconque des revendications précédentes, la surface externe du tube respiratoire (110) étant munie d'un moyen de renforcement (118).
